# EUROPÄISCHE PATENTSCHRIFT

(11) **EP 3 452 173 B1**
(45) Veröffentlichungstag und Bekanntmachung des Hinweises auf die Patenterteilung: **23.10.2019**
(21) Anmeldenummer: 17715719.5
(22) Anmeldetag: 05.04.2017
(51) Int. Cl.: A61Q 5/12, A61K 8/86, A61K 8/898, A61K 8/39

(54) **WÄSSRIGE EMULSIONEN ENTHALTEND AMINOFUNKTIONELLE ORGANOPOLYSILOXANE UND NICHT-IONISCHE EMULGATOREN**
AQUEOUS EMULSIONS CONTAINING AMINO-FUNCTIONAL ORGANOPOLYSILOXANES AND NON-IONIC EMULSIFIERS
ÉMULSIONS AQUEUSES CONTENANT DES ORGANOPOLYSILOXANES À FONCTION AMINO ET DES ÉMULSIFIANTS NON IONIQUES

(30) Priorität: 03.05.2016 DE 102016207603
(43) Veröffentlichungstag der Anmeldung: 13.03.2019
(73) Patentinhaber: Wacker Chemie AG, 81737 München (DE)
(72) Erfinder: RAUTSCHEK, Holger, 01612 Nünchritz (DE); KAUSCHKE, Marco, 01612 Glaubitz (DE)
(74) Vertreter: Deffner-Lehner, Maria
(86) Internationale Anmeldenummer: PCT/EP2017/058070
(87) Internationale Veröffentlichungsnummer: WO 2017/190906

(56) Entgegenhaltungen:
- EP-A1- 0 646 618
- WO-A1-2017/106404
- DATABASE GNPD [Online] MINTEL; 1. April 2016 (2016-04-01), "Shampoo Refresh", XP002772134, Database accession no. 3965797
- Anonymous: "BASF Surfactants", , 30. April 2013 (2013-04-30), XP055390863, Gefunden im Internet: URL:https://biokhim.com/data2/basf/BASF Surfactants.pdf [gefunden am 2017-07-14]
- Anonymous: "Thieme RÖMPP Emulgatoren", , 1. April 2017 (2017-04-01), XP055390654, Gefunden im Internet: URL:https://roempp.thieme.de/roempp4.0/do/ data/RD-05-00974 [gefunden am 2017-07-13]
- Anonymous: "Thieme RÖMPP Tenside", , 13. Juli 2017 (2017-07-13), XP055390830, Gefunden im Internet: URL:https://roempp.thieme.de/roempp4.0/do/ data/RD-20-00650 [gefunden am 2017-07-14]

## Beschreibung

Die Erfindung betrifft wässrige Emulsionen enthaltend aminofunktionelle Organopolysiloxane und nicht-ionische Emulgatoren und ein Verfahren zu ihrer Herstellung.

Silicone sind vielfältig einsetzbar. Um die Anwendung und Dosierung insbesondere bei viskosen Produkten zu erleichtern, ist es für viele Anwendungen wünschenswert, dass die siliziumorganischen Verbindungen in verdünnter Form vorliegen. Deshalb erfolgt der Einsatz meist in Form von wässrigen Emulsionen oder Dispersionen, üblicherweise als Öl-in-Wasser-Emulsionen (O/W-Emulsionen), die mit Wasser verdünnbar sind. Aus EP 138192 A1 ist bekannt, dass auf der Basis von Organopolysiloxanen mit polaren Gruppen besonders feinteilige Emulsionen, sogenannte Mikroemulsionen, die transluzent bis klar sind, hergestellt werden können. Derartige Emulsionen sind insbesondere dort vorteilhaft einsetzbar, wo die herausragende Stabilität dieser Emulsionen benötigt wird, z.B. bei der Textilbehandlung, wo Ölflecken vermieden werden müssen oder wenn z.B. klare kosmetische Formulierungen hergestellt werden sollen.

Als Emulgatoren für die Herstellung dieser Microemulsionen werden überwiegend nichtionogene Emulgatoren insbesondere ethoxylierte Alkohole vorgeschlagen. Beispiele sind ethoxyliertes Trimethylnonanol (z.B. EP 299596 B1, EP 943644 B1), ethoxylierte Isotridecyl- (z.B. EP 442098 B1, EP 515915 B1 und EP 859029 A2), ethoxylierte Isodecylalkohole (z.B. EP 1560972 B1), Mischungen aus ethoxylierten Isotridecylalkoholen und ethoxylierten Oxoalkoholen (also verzweigten Alkoholen) mit 10 Kohlenstoffatomen (DE 19835227 A1), und ethoxylierte Fettalkohole wie n-Dodecanol (z.B. EP 475363 A2). Zusätzlich zu den Emulgatoren können die Mikroemulsionen Lösungsmitteln, wie z.B. Alkohole (z.B. EP 417559 B1), oder Glycole, wie Monoethylenglycolhexylether, Monoethylenglycolbutylether, Diethylenglycolhexylether, Diethylenglycolbutylether (z.B. WO15013247 A1), oder Polyole (z.B. EP 0859029 A2) enthalten. Diese Mikroemulsionen enthalten meist maximal 20% an Siloxan mit polaren Gruppen (z.B. EP 2215148 B1). Bei höheren Anteilen steigt die Viskosität stark an, so dass die Handhabung der Produkte schwer bis unmöglich wird (vgl. DE 19835227 A1 S. 3 Z. 28-34).

DE 19835227 A1 offenbart, dass durch Abmischen des aminofunktionellen Organopolysiloxans mit unsubstituierten Polydimethylsiloxan und Zugabe von Polyethylenglycol es möglich ist, klare Produkte mit über 30% Siloxananteil zu erhalten, ansonsten wird ein festes Gel gebildet. Der Zusatz von Salz zu diesem Gel, reduzierte die Viskosität, aber auch die Stabilität, der Zusatz von Isopropanol führte zu einem Produkt mit einem niedrigen Flammpunkt, was ebenfalls unerwünscht ist.

Die Viskosität kann auch durch den Zusatz der oben erwähnten Glycole nur begrenzt beherrscht werden. So sind z.B. zusätzlich zu 10% Emulgator weitere 15% Butyldiglycol erforderlich, um eine handhabbare Mikroemulsion mit 30% aminomodifizierten Organopolysiloxan zu erhalten (EP 646618 B1), also über 80% Emulgatoren/Glycole bezogen auf das Organopolysiloxan. Dieser hohe Anteil an Bestandteilen, die keinen Beitrag zur Wirksamkeit des Produktes bei der Anwendung leisten, aber entsprechende Kosten verursachen und letztlich auch die Umwelt unnötig belasten, ist nicht erwünscht.

Es bestand die Aufgabe, transluzente bis klare Emulsionen mit kleiner Partikelgröße auf der Basis von aminofunktionellen Organopolysiloxanen bereitzustellen, die auch bei einem hohen Gehalt an Organopolysiloxan gut handhabbar und lagerstabil sind und keine Lösungsmittel enthalten.

Die Aufgabe wird durch die Erfindung gelöst.

Gegenstand der Erfindung sind wässrige Emulsionen enthaltend
(A) aminofunktionelle Organopolysiloxane, die Si-C-gebundene Reste mit basischem Stickstoff enthalten,
(B) gegebenenfalls Organopolysiloxane, die von den aminofunktionellen Organopolysiloxanen (A) verschieden sind,
(C) nicht-ionische Emulgatoren,
(D) gegebenenfalls ionische Emulgatoren,
(E) gegebenenfalls Hilfsstoffe und
(F) Wasser,
dadurch gekennzeichnet, dass als nicht-ionische Emulgatoren (C) Polyethylenglycolether von linearen primären Alkoholen, die lineare Kohlenwasserstoffreste mit höchstens 10 Kohlenstoffatomen aufweisen, eingesetzt werden,
mit der Maßgabe, dass die Mitverwendung von Polyethylenglycolethern von primären Alkoholen, die verzweigte Kohlenwasserstoffreste aufweisen, und die Mitverwendung von Polyethylenglycolethern von sekundären Alkoholen ausgeschlossen ist.

Organopolysiloxane (A) sind vorzugsweise Organopolysiloxane enthaltend Einheiten der Formel

Rₐ(R²O)_{b}Q_{c}SiO_{(4-a-b-c)/2} (I),

worin
R gleich oder verschieden ist und einen einwertigen, gegebenenfalls substituierten Kohlenwasserstoffrest mit 1 bis 30 Kohlenstoffatomen oder ein Wasserstoffatom bedeutet,
R² gleich oder verschieden ist und ein Wasserstoffatom oder einen einwertigen, gegebenenfalls substituierten Kohlenwasserstoffrest mit 1 bis 20, vorzugsweise 1 bis 6 Kohlenstoffatomen, bedeutet,
Q einen mit Aminogruppen substituierten Rest der Formel

   -R³-[NR⁴-R⁵-]ₓNR⁴₂ (II)

   bedeutet, worin
   R³ gleich oder verschieden ist und einen zweiwertigen, Si-C-gebundenen Kohlenwasserstoffrest mit 1 bis 18 C-Atomen bedeutet,
   R⁴ ein Wasserstoffatom oder einen einwertigen linearen, cyclischen oder verzweigten, gesättigten oder ungesättigten Kohlenwasserstoffrest mit 1-18 Kohlenstoffatomen bedeutet,
   R⁵ gleich oder unterschiedlich ist und einen zweiwertigen Kohlenwasserstoffrest mit 1 bis 6 C-Atomen bedeutet,
   x 0 oder eine ganze Zahl von 1 bis 10, vorzugsweise 0 oder 1, ist,
   a 0, 1, 2 oder 3 ist,
   b 0, 1, 2 oder 3, vorzugsweise 0 oder 1 ist, und
   c 0, 1, 2 oder 3, vorzugsweise 0 oder 1 ist,
   mit der Maßgabe, dass die Summe a+b+c kleiner oder gleich 3 ist.

Vorzugsweise hat die Summe a+b+c einen durchschnittlichen Wert von 1,5 bis 2,5, bevorzugt 1,9 bis 2,1.

Beispiele für unsubstituierte Kohlenwasserstoffreste R sind Alkylreste, wie der Methyl-, Ethyl-, n-Propyl , iso-Propyl-, 1-n-Butyl-, 2-n-Butyl-, iso-Butyl-, tert.-Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Octadecylreste, wie der n-Octadecylrest; Cycloalkylreste, wie der Cyclopentyl-, Cyclohexyl-, Cycloheptylrest und Methylcyclohexylreste; Alkenylreste, wie der Vinyl-, 1-Propenyl- und der 2-Propenylrest; Arylreste, wie der Phenyl-, Naphthyl-, Anthryl- und Phenanthrylrest; Alkarylreste, wie o-, m-, p-Tolylreste; Xylylreste und Ethylphenylreste; und Aralkylreste, wie der Benzylrest, der der α- und der β-Phenylethylrest.

Beispiele für substituierte Reste R sind mit Halogen-, Cyano-, Glycidoxy- oder Polyalkylenglycol- substituierte Kohlenwasserstoffreste, wie beispielsweise Trifluorpropyl-, Cyanoethyl-, Glycidoxypropyl-, Polyalkylenglycolpropylreste.

Als Kohlenwasserstoffreste R sind Methyl- und Phenylreste bevorzugt.

Beispiele für Reste R² sind das Wasserstoffatom, Methyl-, Ethyl-, Propyl- und Butylreste. Vorzugsweise ist der Rest R² ein Wasserstoffatom, ein Ethyl- oder ein Methylrest.

Beispiele für mit Aminogruppen substituierte Reste Q sind:

H₂N(CH₂)₂NH(CH₂)₃-

H₂N(CH₂)₃-

H₂N(CH₂)₂NH-CH₂-CH(CH₃)-CH₂-

H₂N-CH₂-CH(CH₃)-CH₂-

H₃CNH(CH₂)₃-

H₂N(CH₂)₄-

H₂N(CH₂)₅-

H(NHCH₂CH₂)₃-

C₄H₉NH(CH₂)₂NH(CH₂)₂-

und

cyclo-C₆H₁₁NH(CH₂)₃-,

wobei

H₂N(CH₂)₃-

und

H₂N(CH₂)₂NH(CH₂)₃-

besonders bevorzugt sind.

Bei den Organopolysiloxanen (A), welche in dem erfindungsgemäßen Verfahren verwendet werden, handelt es sich vorzugsweise um solche enthaltend 5 bis 10000 insbesondere 50-1000 Einheiten der Formel (I), besonders bevorzugt um solche aus Einheiten der Formel (I) mit einem durchschnittlichen Wert von a von 1,90 bis 2,3, einem durchschnittlichen Wert von b von 0 bis 0,2 einem durchschnittlichen Wert von c von 0,002 bis 0,1.

Vorzugsweise enthalten die Organopolysiloxane (A) außer den Aminogruppen substituierte Reste R² keine weiteren substituierten Kohlenwasserstoffreste.

Bevorzugt als Organopolysiloxane (A) sind solche der Formel

R*_{3-y}Q_{y}SiO[R₂SiO]ₖ[RQSiO]ₗSiQ_{y}R*_{3-y} (III),

wobei
- R* R: oder einen Rest der Formel -OR² bedeutet,
- R, R²: und Q die oben dafür angegebene Bedeutung haben,
- k: eine ganze Zahl von 10 bis 1000 ist,
- l: eine ganze Zahl von 1 bis 100 ist und
- y: 0 oder 1 ist, vorzugsweise 0, ist.

Vorzugsweise haben die Organopolysiloxane (A) eine Viskosität von 50 mm²/s bis 100000 mm²/s gemessen bei 25°C nach DIN 53019.

Vorzugsweise haben die Organopolysiloxane (A) einen Gehalt an basischen Stickstoff von 0,05 mmol/g bis 2,0 mmol/g besonders bevorzugt 0,15 mmol/g bis 1,0 mmol/g.

Gegebenenfalls mitverwendete Organopolysiloxane (B) bestehen vorzugsweise aus Einheiten der Formel

R⁸ₑ(R⁹O)_{f}SiO_{(4-e-f)/2} (IV),

wobei
R⁸ gleich oder verschieden ist und einen einwertigen, gegebenenfalls substituierten Kohlenwasserstoffrest mit 1 bis 30 Kohlenstoffatomen oder ein Wasserstoffatom bedeutet,
R⁹ gleich oder verschieden ist und ein Wasserstoffatom oder einen einwertigen, gegebenenfalls substituierten Kohlenwasserstoffrest mit 1 bis 20, vorzugsweise 1 bis 6 Kohlenstoffatomen, bedeutet,
e 0, 1, 2 oder 3 ist und
f 0, 1, 2 oder 3, vorzugsweise 0 oder 1 ist.

Beispiele für Reste R gelten im vollen Umfang für Reste R⁸.

Beispiele für Reste R² gelten im vollen Umfang für Reste R⁹.

Vorzugsweise handelt es sich bei den Organopolysiloxanen (B) um Polydimethylsiloxane mit Trimethylsilylendgruppen oder Dimethylhydroxysilylendgruppen.

Vorzugsweise haben die Organopolysiloxane (B) eine Viskosität von 5 mm²/s - 1000 mm²/s gemessen bei 25°C nach DIN 53019.

Vorzugsweise ist die Mitverwendung von Organopolysiloxanen der Formel

R¹(CH₃)₂SiO[(CH₃)₂SiO]ₙSi(CH₃)₂R¹ (V),

wobei R¹ einen Hydroxy-, Methoxy-, Ethoxy- oder Methylrest und n 25 bis 900 bedeutet,
in Mengen von größer oder gleich 2 Gew.-%, vorzugsweise größer 1 Gew.-%, jeweils bezogen auf das Gesamtgewicht der Emulsion, ausgeschlossen.

Besonders bevorzugt ist die Mitverwendung von Polyorganosiloxanen (B), die von den aminofunktionellen Organopolysiloxanen (A) verschieden sind, in Mengen von größer oder gleich 2 Gew.-%, vorzugsweise größer 1 Gew.-%, jeweils bezogen auf das Gesamtgewicht der Emulsion, ausgeschlossen.

In einer besonders bevorzugten Ausführungsform sind in den erfindungsgemäßen Emulsionen keine Organopolysiloxane (B) enthalten.

Bevorzugt werden als Polyethylenglycolether (C) von linearen primären Alkoholen mit höchstens 10 C-Atomen solche der Formel

CH₃-(CH₂)ₘ-O-(CH₂-CH₂-O)ₚ-R⁶ (VI),

wobei R⁶ ein Wasserstoffatom, einen C₁₋₄-Kohlenwasserstoffrest oder einen Rest der Formel -C(O)R⁷ bedeutet,
R⁷ einen C₁₋₂-Kohlenwasserstoffrest bedeutet
m eine ganze Zahl von 5 bis 9 bedeutet und
p eine ganze Zahl von 3 bis 20 ist,
eingesetzt.
Vorzugsweise ist R⁶ ein Wasserstoffatom.

Beispiele für erfindungsgemäße nicht-ionische Emulgatoren (C) sind Polyethylenglycolether der Formel:

CH₃-(CH₂)₅-O-(CH₂-CH₂-O)₄-H

CH₃-(CH₂)₅-O-(CH₂-CH₂-O)₅-H

CH₃-(CH₂)₇-O-(CH₂-CH₂-O)₄-H

CH₃-(CH₂)₇-O-(CH₂-CH₂-O)₅-H

CH₃-(CH₂)₇-O-(CH₂-CH₂-O)₆-H

CH₃-(CH₂)₇-O-(CH₂-CH₂-O)₈-H

CH₃-(CH₂)₉-O-(CH₂-CH₂-O)₅-H

CH₃-(CH₂)₉-O-(CH₂-CH₂-O)₆-H

CH₃-(CH₂)₉-O-(CH₂-CH₂-O)₁₀-H

CH₃-(CH₂)₉-O-(CH₂-CH₂-O)₁₆-H

CH₃-(CH₂)₉-O-(CH₂-CH₂-O)₂₀-H

Vorzugsweise ist die Mitverwendung von Polyethylenglycolethern von verzweigten primären Alkoholen, also solche die verzweigte Kohlenwasserstoffreste aufweisen, und die Mitverwendung von Polyethylenglycolethern von sekundären Alkoholen ausgeschlossen.

Die erfindungsgemäßen Emulsionen enthalten vorzugsweise keine (Poly)ethylenglycole, oder (Poly)ethylenglcolether mit Alkoholen mit weniger als 6 Kohlenstoffatomen, Alkylenglycole, Glycerin, einwertige Alkohole mit bis zu 5 Kohlenstoffatomen oder mehrwertige Alkohole mit bis zu 10 Kohlenstoffatomen.

Vorzugsweise enthalten die erfindungsgemäßen Emulsionen keine Emulgatoren auf der Basis von ethoxylierten Alkylphenolen.

Vorzugsweise ist die Mitverwendung von weiteren nicht-ionischen Emulgatoren, die verschieden von den erfindungsgemäßen nicht-ionischen Emulgatoren (C) sind, ausgeschlossen.

Beispiele für gegebenenfalls enthaltene ionische Emulgatoren (D) sind insbesondere kationische Emulgatoren.

Beispiele für kationische Emulgatoren (D) sind alle bekannten quartären Ammoniumverbindungen, die mindestens einen substituierten oder unsubstituierten Kohlenwasserstoffrest mit mindestens 10 Kohlenstoffatomen tragen, wie Dodecyldimethylammoniumchlorid, Tetradecyltrimethylammoniumbromid, Stearyltrimethylammoniumchlorid, Distearyldimethylammoniumchlorid, Cetyltrimethylammoniumchlorid, Behenyltrimethylammoniumbromid, Dedecylbenzyldimethylammoniumchlorid und Benzyltrimethylammoniumchlorid. Es können auch ethoxylierte kationische Emulgatoren eingesetzt werden, wie z.B. Cocosalkylbis(hydroxyethyl)methyl, ethoxyliert, Methylsulfat.

Wenn als Komponente (D) kationische Emulgatoren eingesetzt werden, so sind das bevorzugt Aryl- oder Alkyltrimethylammoniumsalze, wie Stearyltrimethylammoniumchlorid und Cetyltrimethylammoniumchlorid, Benzyltrialkylammoniumsalze, Trimethylbenzylammiumchlorid und Trimethylbenzylammoniummethosulfat.

Weitere Beispiele sind alle bekannten quartären Imidazoliniumverbindungen, die mindestens einen substituierten oder unsubstituierten Kohlenwasserstoffrest mit mindestens 10 Kohlenstoffatomen tragen, wie 1-Methyl-2-stearyl-3-stearylamidoethyl-imidazolinium methosulfat, 1-Methyl-2-norstearyl-3-stearyl-amidoethylimidazolinium-methosulfat, 1-Methyl-2-oleyl-3-oleylamidoethylimidazolinum-methosulfat, 1-Methyl-2-stearyl-3-methylimidazolinum-methosulfat, 1-Methyl-2-behenyl-3-methylimidazolinum-methosulfat und 1-Methyl-2-dodecyl-3-methylimidazolinum-methosulfat.

Als Hilfsstoffe (E) können alle Stoffe eingesetzt werden, die üblicherweise Siliconemulsionen zugesetzt werden und verschieden sind zu den Komponenten (A), (B), (C), (D) und (F), eingesetzt werden, wie z.B. Silane, insbesondere Alkoxysilane, Rheologieadditive, Konservierungsmittel, Mittel zur Einstellung des pH Wertes, Desinfektionsmittel, Netzmittel, Korrosionsinhibitoren, Farbstoffe und Duftstoffe.

Beispiele für Hilfsstoffe (E) sind insbesondere Konservierungsmittel, wie z.B. Benzylalkohol, Phenoxyethanol, Parabene, Salicylsäure, Isothiazolinone und Mittel zur Einstellung des pH Wertes, wie z.B. Ameisensäure, Essigsäure, Propionsäure und deren Salze mit Alkali- oder Erdalkalimetallen oder Aminen.

Als Wasser (F) können alle Arten von Wässer eingesetzt werde, die auch bisher zur Herstellung von Dispersionen eingesetzt wurden.

Als Wasser (F) wird vorzugsweise teil- oder vollentsalztes Wasser, destilliertes oder (mehrfach) redestilliertes Wasser, Wässer für medizinische oder pharmazeutische Zwecke, wie z.B. gereinigtes Wasser (Aqua purificata gemäß Pharm. Eur.), eingesetzt.

Das erfindungsgemäß eingesetzte Wasser (F) hat vorzugsweise eine Leitfähigkeit von weniger als 50 µS/cm, besonders bevorzugt weniger als 10 µS/cm, insbesondere weniger als 1,3 µS/cm, jeweils bei 25°C und 1010 hPa.

Bevorzugt bestehen die erfindungsgemäßen Emulsionen aus
(A) aminofunktionellen Organopolysiloxanen, die Si-C-gebundene Reste mit basischem Stickstoff enthalten,
(C) erfindungsgemäßen Polyethylenglycolethern von linearen primären Alkoholen, die lineare Kohlenwasserstoffreste mit höchstens 10 Kohlenstoffatomen aufweisen,
(E) gegebenenfalls Hilfsstoffen und
(F) Wasser.

Vorzugsweise enthalten die erfindungsgemäßen Emulsionen, bevorzugt bestehen die erfindungsgemäßen Emulsionen aus, 10-60 Gew.-% aminofunktionelle Organopolysiloxane (A) 0-10 Gew.-%, bevorzugt 0-2 Gew.-%, insbesondere 0-1 Gew.-%,
Organopolysiloxane (B), die von (A) verschieden sind, 5-30 Gew.-% erfindungsgemäße nicht-ionische Emulgatoren (C) 0-10 Gew.-% ionische Emulgatoren (D)
0-5 Gew.-% Hilfsstoffe (E) und
20-85 Gew.-% Wasser (F),
bezogen auf das Gesamtgewicht der Emulsionen.

Besonders bevorzugt enthalten die erfindungsgemäßen Emulsionen, insbesondere bestehen die erfindungsgemäßen Emulsionen aus, 25-45 Gew.-% aminofunktionelle Organopolysiloxane (A), 0-5 Gew.-%, bevorzugt 0-2 Gew.-%, insbesondere 0-1 Gew.-%,
Organopolysiloxane (B), die von (A) verschieden sind, 10-20 Gew.-% erfindungsgemäße nicht-ionische Emulgatoren (C) 0-5 Gew.-% ionische Emulgatoren (D)
0-2 Gew.-% Hilfsstoffe (E) und
40-65 Gew.-% Wasser (F)
bezogen auf das Gesamtgewicht der Emulsionen.

In einer bevorzugten Ausführung der Erfindung enthalten die erfindungsgemäßen Emulsionen mindestens 30 Gew.-% und höchstens 40 Gew.-% aminofunktionelle Organopolysiloxane (A).

Vorzugsweise beträgt in den erfindungsgemäßen Emulsionen der Anteil an Emulgatoren (C), (D) und Hilfsstoffen (E) insgesamt weniger als 50 Gew.-%, bezogen auf das Gesamtgewicht der Organopolysiloxanen (A) und ggf. (B).

Die Herstellung der erfindungsgemäßen Emulsionen erfolgt nach an sich bekannten Verfahren. Üblicherweise erfolgt die Herstellung durch einfaches Verrühren aller Bestandteile.

Als Misch- und Homogenisierwerkzeuge, die für die Herstellung der erfindungsgemäßen Emulsionen eingesetzt werden können, sind alle dem Fachmann bekannten Emulgiergeräte, wie beispielsweise schnelllaufende Rührer, Dissolverscheiben, Rotor-Stator-Homogenisatoren, Ultraschallhomogenisatoren und Hochdruckhomogenisatoren verschiedenster Bauart, geeignet. Wenn große Teilchen gewünscht sind, sind auch langsam laufende Rührer geeignet.

Die erfindungsgemäßen Emulsionen können kontinuierlich, semikontinuierlich oder diskontinuierlich hergestellt werden.

Die Herstellung der erfindungsgemäßen Emulsionen erfolgt vorzugsweise bei Temperaturen von 5°C bis 80°C insbesondere 15°C bis 60°C und vorzugsweise beim Druck der umgebenden Atmosphäre. Es kann aber auch Vakuum oder Überdruck eingesetzt werden.

Vorzugsweise werden erst die Öle (A) und (B), die Emulgatoren (C) und (D) sowie die Hilfsstoffe (E) und ggf. maximal 20% der gesamten Menge an Wasser (F) miteinander vermischt und homogenisiert. Anschließend wird dann das Wasser bzw. restliche Wasser langsam bzw. portionsweise eingemischt.

Die erfindungsgemäßen Emulsionen sind bevorzugt klar bis transluzent und weisen Trübungswerte (bei 90°) von bevorzugt <500ppm, besonders bevorzugt <100ppm, insbesondere bevorzugt von <20ppm auf.

Die erfindungsgemäßen Emulsionen haben eine Teilchengröße (Medianwert der Volumenverteilung D(50)) von <100 nm, bevorzugt <50 nm, besonders bevorzugt <10 nm.

Die Viskosität der erfindungsgemäßen Emulsionen bei 25°C ist kleiner als 10000 mPas, bevorzugt kleiner 5000 mPas, besonders bevorzugt kleiner 1000 mPas.

Die erfindungsgemäßen Emulsionen haben einen pH-Wert von bevorzugt 4 bis 8, besonders bevorzugt 4 bis 6.

Die erfindungsgemäßen Emulsionen haben gegenüber dem Stand der Technik den Vorteil, dass sie auch bei hohen Silicongehalten ohne Zugabe von in der Anwendung möglicherweise störenden viskositätssenkenden Additiven, wie z.B. Glycolen, Glycerin oder Salzen, eine niedrige Viskosität aufweisen und eine gute Handhabbarkeit erlauben.

Die erfindungsgemäßen Emulsionen weisen auch mit weniger als 50% Emulgatoren und Additiven, bezogen auf das Silicon (A) und ggf. (B), eine niedrige Teilchengröße, hohe Transparenz bzw. Klarheit und hervorragende Stabilität auf.

Die erfindungsgemäßen Emulsionen können überall dort eingesetzt werden, wo auch bisher Emulsionen auf der Basis von Polyorganosiloxanen mit funktionellen Gruppen, die einen basischen Stickstoff tragen, eingesetzt wurden, z.B. als Textilweichmacher, als Additiv für Waschmittel, als Trennmittel und in Haarpflegemitteln wie Conditioner und Shampoos. Gegenstand der Erfindung sind kosmetische Formulierungen, insbesondere Haarbehandlungsmittel, enthaltend die erfindungsgemäßen wässrigen Emulsionen.

Gegenstand der Erfindung sind weiterhin Wasch- und Reinigungsmittel enthaltend die erfindungsgemäßen wässrigen Emulsionen.

Die in den nachfolgenden Beispielen hergestellten Emulsionen wurden wie folgt geprüft:
Die Teilchengröße wurde mit einem Partikelgrößenanalysator Zetasizer ZEN1600/ Nano-S, Fa. Malvern, Software Version 6.01 mittels dynamischer Lichtstreuung bestimmt. Dazu wurden die Emulsionen mit gefilterten und entgasten Wasser auf 0,5% verdünnt. Die angegebenen Werte beziehen sich immer auf den Wert D(50), dieser ist der Medianwert der Volumenverteilung, wie in Basic Principles of Particle Size Analysis, Technical Paper, von Dr. Alan Rawle, Malvern Instruments Limited 2000, beschrieben. Folgende Parameter wurden verwendet: Material RI 1,39, Dispersant RI 1,33, Material Absorption 0,010, Viskosität 0,8872 mPas, Temperatur 25°C, Count rate 159,6, Zeitdauer 50 s, Messposition 4,65 mm.

Die Trübung der Emulsionen wurde entsprechend bei 25°C mit einem LabScat 2-Winkel Trübungsmessgerät der Fa. Sigrist in einer Weithalsglasflasche (250mL, d=68mm, h=115mm) im Winkel von 90° bestimmt.

Die Viskositäten der Emulsionen wurden nach DIN EN ISO 2555 mit einem "Brookfield programable Viskosimeter DV-II+" mit Spindel 3 bis Spindel 7 bei 20°C und 20 Umdrehungen/min gemessen, der Wert wurde nach 20s abgelesen.

Die Messung des pH-Wertes erfolgte entsprechend der US Pharmacopeia USP 33 bei 20°C.

Die Aminzahl gibt an wieviel mmol Aminogruppen je g des Siloxans A enthalten sind. Die Aminzahl kann durch potentiometrische Titration der durch Protonierung mit Eisessig entstandenen Salze mit Perchlorsäure bestimmt werden.

Folgende Stoffe wurden verwendet:
**Siloxan A1:** Ein reaktives (Hydroxy/Methoxy-endständiges) Aminoethylaminopropylfunktionelles Polydimethylsiloxan mit einer Viskosität von 1000 mPas bei 25°C und einer Aminzahl vom 0,3 mmol/g, erhältlich unter der Bezeichnung Finish WR 1300 bei der Wacker Chemie AG, D-München
**Siloxan A2:** Ein mit Trimethylsiloxygruppen terminiertes Aminoethylaminopropylfunktionelles Polydimethylsiloxan mit einer Viskosität von 1000 mPas bei 25°C und einer Aminzahl vom 0,6 mmol/g, erhältlich unter der Bezeichnung Finish WT 1650 bei der Wacker Chemie AG, D-München
**Emulgator C1** n-Decylpolyethylenglycolether mit durchschnittlich 6 Ethylenglycolgruppen erhältlich unter dem Namen Aduxol 10 D 06 bei Chemische Fabrik Schärer & Schläpfer AG, CH-Rothrist
**Emulgator C2** n-Decylpolyethylenglycolether mit durchschnittlich 5 Ethylenglycolgruppen erhältlich unter dem Namen Aduxol 10D 05 bei Chemische Fabrik Schärer & Schläpfer AG, CH-Rothrist
**Emulgator C3** n-Hexylpolyethylenglycolether mit durchschnittlich 5 Ethylenglycolgruppen erhältlich unter dem Namen Emulan HE 50 bei der BASF SE, D-Ludwigshafen.
**Emulgator C4:** n-Octylpolyethylenglycolether mit durchschnittlich 4 Ethylenglycolgruppen erhältlich unter dem Namen Dehydol 04 DEO bei der BASF SE, D-Ludwigshafen.
**Emulgator CV1:** (nicht erfindungsgemäß) iso-Decylpolyethylenglycolether mit durchschnittlich 6 Ethylenglycolgruppen erhältlich unter dem Namen Aduxol DEC 06 bei Chemische Fabrik Schärer & Schläpfer AG, CH-Rothrist
**Emulgator CV2:** (nicht erfindungsgemäß) iso-Decylpolyethylenglycolether mit durchschnittlich 5 Ethylenglycolgruppen erhältlich unter dem Namen LUTENSOL® XP 50 bei der BASF SE, D-Ludwigshafen
**Emulgator CV3:** (nicht erfindungsgemäß) iso-Tridecylpolyethylenglycolether mit durchschnittlich 6 Ethylenglycolgruppen erhältlich unter dem Namen IMBENTIN® T060 bei der KOLB AG, CH-Hedingen
**Emulgator CV4:** (nicht erfindungsgemäß) n-Dodecylpolyethylenglycolether mit durchschnittlich 4 Ethylenglycolgruppen erhältlich unter dem Namen SYMPATENS® ALM/040 bei der KOLB AG, CH-Hedingen
**Emulgator CV5:** (nicht erfindungsgemäß) n-Dodecylpolyethylenglycolether mit durchschnittlich 23 Ethylenglycolgruppen erhältlich unter dem Namen SYMPATENS® ALM/230 G bei der KOLB AG, CH-Hedingen
**Emulgator D1:** Hexadecyltrimethylammoniumchlorid, erhältlich als 30%ige wässrige Lösung unter der Bezeichnung Genamin® CTAC bei der Clariant GmbH, D-Frankfurt/Main
**Emulgator D2:** Cocosalkylbis(hydroxyethyl)methyl, ethoxyliert, Methylsulfat erhältlich unter der Bezeichnung SERVAMINE® KW 100 bei Elementis Specialties Netherlands B.V., NL-Delden.
**Hilfsstoff E1:** Essigsäure Pharm. Eur., erhältlich bei der CSC Jäklechemie, D-Nürnberg, zum Einstellen des pH-Wertes
**Hilfsstoff E2:** Phenoxyethanol kosmetische Qualität, erhältlich bei der Schülke&Mayr GmbH, D-Norderstedt, als Konservierungsmittel

### Beispiel 1:

122,5g Siloxan A1, 49g Emulgator C1, 0,7g Essigsäure (E1) und 3,15g Phenoxyethanol (E2) werden mit einem ULTRA-TURRAX® T50 1 min bei 4000/min vermischt. Anschließend werden innerhalb von 3 min 90g entionisiertes Wasser zugegeben und weitere 5 min bei 4000/min homogenisiert. Die entstandene pastöse Emulsion wurde innerhalb von 5 min mit 85,1g Wasser verdünnt und 4h bei 60°C gelagert. Es wurde eine klare fließfähige Emulsion erhalten. Diese Emulsion war nach 28d bei 50°C und nach einem Jahr Lagerung bei Raumtemperatur völlig stabil.

### Vergleichsbeispiel V1:

Beispiel 1 wird wiederholt aber statt Emulgator C1 wird Emulgator CV 1 verwendet. Es wird ein festes Gel erhalten, was auch nach 4h bei 60°C noch nicht fließfähig war.

### Vergleichsbeispiel V2:

Das Produkt von Vergleichsbeispiel V1 wird 16h bei 80°C gelagert. Die Mischung war jetzt dünnflüssig aber trüb. Nach 28d bei 50°C war die Emulsion getrennt.

### Vergleichsbeispiel V3:

Beispiel 1 wird wiederholt aber statt Emulgator C1 wird Emulgator CV 2 verwendet. Es wird ein festes Gel erhalten, was auch nach 4h bei 60°C noch sehr viskos war.

### Vergleichsbeispiel V4:

Beispiel 1 wird wiederholt aber statt Emulgator C1 wird Emulgator CV 3 verwendet. Es wird ein festes Gel erhalten, was auch nach 4h bei 60°C noch sehr viskos war.

### Vergleichsbeispiel V5:

Beispiel 1 wird wiederholt aber statt 49g Emulgator C1 werden 32,66g Emulgator CV 4 und 16,33g Emulgator CV 5 verwendet. Es wird ein festes Gel erhalten, was auch nach 4h bei 60°C noch fest war.

### Beispiel 2:

122,5g Siloxan A2, 49g Emulgator C1, 0,7g Essigsäure (E1) und 3,15g Phenoxyethanol (E2) werden mit einem ULTRA-TURRAX® T50 1 min bei 4000/min vermischt. Anschließend werden innerhalb von 3 min 80g entionisiertes Wasser zugegeben und weitere 5 min bei 4000/min homogenisiert. Die entstandene pastöse Emulsion wurde innerhalb von 5 min mit 95,1g Wasser verdünnt. Es wurde eine klare niedrigviskose Emulsion erhalten.

### Beispiel 3:

140g Siloxan A1, 56g Emulgator C1, 4,0g Emulgator D1, 0,8g Essigsäure (E1) und 3,6g Phenoxyethanol (E2) werden mit einem ULTRA-TURRAX® T50 1 min bei 4000/min vermischt. Die entstandene pastöse Emulsion wurde innerhalb von 5 min mit 145,6g Wasser verdünnt. Es wurde eine klare fließfähige Emulsion erhalten.

### Vergleichsbeispiel V6:

Beispiel 4 wird wiederholt aber statt Emulgator C1 wird Emulgator CV 1 verwendet. Es wird eine viskose Emulsion erhalten, die auch nach 4h bei 60°C noch sehr viskos war.

### Beispiel 4:

122,5g Siloxan A1, 49g Emulgator C1, 1,15g Emulgator D2, 0,7g Essigsäure (E1) und 3,15g Phenoxyethanol (E2) werden mit einem ULTRA-TURRAX® T50 1 min bei 4000/min vermischt. Anschließend werden innerhalb von 3 min 173,5g entionisiertes Wasser zugegeben und weitere 5 min bei 4000/min homogenisiert. Es wurde eine klare fließfähige Emulsion erhalten

### Beispiel 5:

122,5g Siloxan A1, 24,5g Emulgator C1, 24,5g Emulgator C3, 0,7g Essigsäure (E1) und 3,15g Phenoxyethanol (E2) werden mit einem ULTRA-TURRAX® T50 1 min bei 4000/min vermischt. Anschließend werden innerhalb von 3 min 174,65g entionisiertes Wasser zugegeben und weitere 5 min bei 4000/min homogenisiert. Es wurde eine fast klare dünnflüssige Emulsion erhalten.

### Beispiel 6:

122,5g Siloxan A2, 24,5g Emulgator C2, 24,5g Emulgator C3, 0,7g Essigsäure (E1) und 3,15g Phenoxyethanol (E2) werden mit einem ULTRA-TURRAX® T50 1 min bei 4000/min vermischt. Anschließend werden innerhalb von 3 min 174,65g entionisiertes Wasser zugegeben und weitere 5 min bei 4000/min homogenisiert. Es wurde eine fast klare fließfähige Emulsion erhalten.

### Beispiel 7:

122,5g Siloxan A1, 49,0g Emulgator C4, 3,5g Emulgator D1, 0,7g Essigsäure (E1) und 3,15g Phenoxyethanol (E2) werden mit einem ULTRA-TURRAX® T50 1 min bei 4000/min vermischt. Anschließend werden innerhalb von 3 min 171,15g entionisiertes Wasser zugegeben und weitere 5 min bei 4000/min homogenisiert. Es wurde eine fast klare dünnflüssige Emulsion erhalten.

### Beispiel 8:

122,5g Siloxan A2, 49,0g Emulgator C2, 0,7g Essigsäure (E1) und 3,15g Phenoxyethanol (E2) werden mit einem ULTRA-TURRAX® T50 1 min bei 4000/min vermischt. Anschließend werden innerhalb von 3 min 174,65g entionisiertes Wasser zugegeben und weitere 5 min bei 4000/min homogenisiert. Es wurde eine fast klare dünnflüssige Emulsion erhalten.

### Vergleichsbeispiel V7:

Beispiel 9 wird wiederholt aber statt Emulgator C2 wird Emulgator CV 2 verwendet. Es wird eine trübe viskose Emulsion erhalten.

**Tabelle 1: Eigenschaften der in den Beispielen erhaltenen Produkte**

| Beispiel | Siloxananteil / Emulgatoren in Gew.-% | Aussehen | Viskosität in mPas bei 25°C | D (50) in nm | Trübung bei 90° in ppm |
|---|---|---|---|---|---|
| 1 | 35 / 14 | klar | 1500 | 5,4 | 70,2 |
| V1 | 35 / 14 | festes Gel | - | - | - |
| V2 | 35 / 14 | trüb | 970 | Nicht messbar | >1000 |
| V3 | 35 / 14 | klar | 11680 | 3,3 | 16,4 |
| V4 | 35 / 14 | klar | 17080 | 3,6 | 21,9 |
| V5 | 35 / 14 | festes Gel | - | - | - |
| 2 | 35 / 14 | fast klar | 360 | 35,0 | 68,6 |
| 3 | 40 / 16,34 | klar | 2190 | 10,3 | 53,3 |
| V6 | 40 / 16,34 | sehr viskos, trüb | 15160 | 48,2 | 750 |
| 4 | 35 / 14,3 | fast klar | 610 | 45,0 | 80,6 |
| 5 | 35 / 14 | klar | 980 | 5,9 | 48,9 |
| 6 | 35 / 14 | fast klar | 190 | 26,8 | 115 |
| 7 | 34 / 14,3 | fast klar | 130 | 36,5 | 106 |
| 8 | 34 / 14 | leicht trüb | 1010 | 10,4 | 365 |
| V7 | 34 / 14 | viskos, trüb | 430 | 37,5 | >1000 |

Die nicht-erfindungsgemäßen Emulsionen sind schwer handhabbar, denn sie ergeben wie V1 oder V5 ein festes Gel, oder sind wie V3, V4 und V6 sehr viskos oder sind wie V2, V6 und V7 trüb.

Die erfindungsgemäßen Emulsionen sind dagegen gut handhabbar, denn sie haben eine niedrige Viskosität, sind klar und nicht trüb.

### Anwendungsbeispiel A1:

Ein Conditioner wird wie folgt formuliert, wobei die einzelnen Komponenten entsprechend der INCI-Nomenklatur bezeichnet werden:
87,04 Teile Wasser werden vorgelegt und unter Rühren auf 75°C erwärmt. Dabei werden 1,2 Teile Hydroxyethylcellulose (erhältlich unter dem Namen Tylose® H 4000 P2 bei der Shin-Etsu Chemical Co., J-Tokyo) zugegeben. Wenn 65°C erreicht sind, werden 0,5 Teile Stearamidopropyl Dimethylamin (erhältlich unter dem Namen Incromine™ SB bei der Croda GmbH, D-Nettetal), 1 Teil Polysorbate 80 (erhältlich unter dem Namen Tween™ 80 bei der Croda GmbH, D-Nettetal), 3 Teile Stearyl Alcohol (erhältlich unter dem Namen Stearylalkohol bei der Merck-Schuchardt, D-Hohenbrunn), 1 Teil Cetyl Alcohol (erhältlich unter dem Namen Cetylalkohol bei der Merck KGaA, D-Grafing) und 1,76 Teile Behentrimonium Chlorid (erhältlich unter dem Namen Genamin® KDMP bei der Clariant GmbH, D-Frankfurt/Main) zugegeben. Die Mischung wird gemischt bis 75°C erreicht sind. Dann wird die Mischung abgekühlt. Während des Abkühlens werden 0,2 Teile Citric Acid (erhältlich unter dem Namen Citric Acid bei Sigma-Aldrich Chemie GmbH, D-Taufkirchen) und 0,2 Teile Tetrasodium EDTA (erhältlich unter dem Namen EDETA® B Pulver bei der BASF SE, D-Ludwigshafen) zugegeben. Wenn 35°C erreicht sind, werden 0,1 Teile Konservierungsmittel Methylchloroisothiazolinon/Methylisothiazolinon (erhältlich unter dem Namen MICORCARE® IT bei der Thor GmbH Speyer), 4 Teile einer Dimethiconolemulsion (Erhältlich unter dem Namen BELSIL ® DM 3112 VP bei der Wacker Chemie AG, München) und 2 Teile der Emulsion von Beispiel 1 zugegeben und 5 Minuten gerührt. Abschließend wird eine Minute mit einem ULTRA-TURRAX® T50 homogenisiert.

Der so erhaltene Conditioner verbessert sowohl die Trocken- und die Nasskämmbarkeit als auch das Griffgefühl im nassen und trockenen Haar.

### Anwendungsbeispiel A2:

Die Emulsion von Beispiel 5 wird auf 0,5% Silicongehalt verdünnt. Ein Baumwollgewebe wird in diese Flotte getaucht und durch Abquetschen wird eine Flüssigkeitsaufnahme von ca. 75 Gew.-%, bezogen auf das Gewicht der Baumwolle, eingestellt. Anschließend wurde das Gewebe 5 min bei 150°C getrocknet. Das Gewebe hatte anschließend einen hervorragenden Weichgriff.

## Patentansprüche

1. Wässrige Emulsionen bestehend aus
(A) 25-45 Gew.-% aminofunktionelle Organopolysiloxane, die Si-C-gebundene Reste mit basischem Stickstoff enthalten,
(B) 0-5 Gew.-% Organopolysiloxane, die von den aminofunktionellen Organopolysiloxanen (A) verschieden sind,
(C) 10-20 Gew.-% nicht-ionische Emulgatoren,
(D) 0-5 Gew.-% ionische Emulgatoren,
(E) 0-2 Gew.-% Hilfsstoffe und
(F) 40-65 Gew.-% Wasser,
wobei als nicht-ionische Emulgatoren (C) Polyethylenglycolether von linearen primären Alkoholen, die lineare Kohlenwasserstoffreste mit höchstens 10 Kohlenstoffatomen aufweisen, der Formel
CH₃-(CH₂)ₘ-O-(CH₂-CH₂-O)ₚ-R⁶ (VI),
wobei R⁶ ein Wasserstoffatom oder einen C₁₋₄-Kohlenwasserstoffrest bedeutet,
m eine ganze Zahl von 5 bis 9 bedeutet und
p eine ganze Zahl von 3 bis 20 ist,
eingesetzt werden,
mit der Maßgabe, dass die Mitverwendung von Polyethylenglycolethern von primären Alkoholen, die verzweigte Kohlenwasserstoffreste aufweisen, und von Polyethylenglycolethern von sekundären Alkoholen ausgeschlossen ist.

2. Wässrige Emulsionen nach Anspruch 1, **dadurch gekennzeichnet, dass** als aminofunktionelle Organopolysiloxane (A) solche der Formel
R*_{3-y}Q_{y}SiO[R₂SiO]ₖ[RQSiO]ₗSiQ_{y}R*_{3-y} (III),
wobei
R* R oder einen Rest der Formel -OR² bedeutet,
R gleich oder verschieden ist und einen einwertigen, gegebenenfalls substituierten Kohlenwasserstoffrest mit 1 bis 30 Kohlenstoffatomen oder ein Wasserstoffatom bedeutet,
R² gleich oder verschieden ist und ein Wasserstoffatom oder einen einwertigen, gegebenenfalls substituierten Kohlenwasserstoffrest mit 1 bis 20 Kohlenstoffatomen bedeutet,
Q einen mit Aminogruppen substituierten Rest der Formel
-R³-[NR⁴-R⁵-]ₓNR⁴₂ (II)
bedeutet, worin
R³ gleich oder verschieden ist und einen zweiwertigen, Si-C-gebundenen Kohlenwasserstoffrest mit 1 bis 18 C-Atomen bedeutet,
R⁴ ein Wasserstoffatom oder einen einwertigen linearen, cyclischen oder verzweigten, gesättigten oder ungesättigten Kohlenwasserstoffrest mit 1-18 Kohlenstoffatomen bedeutet,
R⁵ gleich oder unterschiedlich ist und einen zweiwertigen Kohlenwasserstoffrest mit 1 bis 6 C-Atomen bedeutet,
x 0 oder eine ganze Zahl von 1 bis 10 ist,
k eine ganze Zahl von 10 bis 1000 ist,
l eine ganze Zahl von 1 bis 100 ist und
y 0 oder 1 ist, vorzugsweise 0, ist,
eingesetzt werden.

3. Wässrige Emulsionen nach Anspruch 1 oder 2, **dadurch gekennzeichnet, dass** (D) kationische Emulgatoren mitverwendet werden.

4. Wässrige Emulsionen nach Anspruch 1, 2 oder 3, **dadurch gekennzeichnet, dass** als Hilfsstoffe (E) Konservierungsmittel, Mittel zur Einstellung des pH-Wertes, Silane, Rheologieadditive, Desinfektionsmittel, Netzmittel, Korrosionsinhibitoren, Farbstoffe oder Duftstoffe eingesetzt werden.

5. Verwendung der wässrigen Emulsionen nach einem der Ansprüche 1 bis 4 zur Herstellung von kosmetischen Formulierungen, insbesondere Haarbehandlungsmitteln.

6. Verwendung der wässrigen Emulsionen nach einem der Ansprüche 1 bis 4 zur Herstellung von Wasch- und Reinigungsmitteln.

## Claims

1. Aqueous emulsions consisting of
(A) 25-45% by weight of amino-functional organopolysiloxanes containing Si-C-bonded radicals comprising basic nitrogen,
(B) 0-5% by weight of organopolysiloxanes which are distinct from the amino-functional organopolysiloxanes (A),
(C) 10-20% by weight of nonionic emulsifiers,
(D) 0-5% by weight of ionic emulsifiers,
(E) 0-2% by weight of auxiliaries and
(F) 40-65% by weight of water,
wherein as nonionic emulsifiers (C) polyethylene glycol ethers of linear primary alcohols comprising linear hydrocarbon radicals having not more than 10 carbon atoms of formula
CH₃-(CH₂)ₘ-O-(CH₂-CH₂-O)ₚ-R⁶ (VI),
wherein R6 represents a hydrogen atom or a C1-4-hydrocarbon radical,
m represents an integer from 5 to 9 and
p is an integer from 3 to 20,
are employed,
with the proviso that the co-use of polyethylene glycol ethers of primary alcohols comprising branched hydrocarbon radicals and of polyethylene glycol ethers of secondary alcohols is excluded.

2. Aqueous emulsions according to Claim 1, **characterized in that** as amino-functional organopolysiloxanes (A) those of formula
R*_{3-y}Q_{y}SiO[R₂SiO]ₖ[RQSiO]ₗSiQ_{y}R*_{3-y} (III),
wherein
R* represents R or a radical of formula -OR²,
R is identical or different and represents a monovalent, optionally substituted hydrocarbon radical having 1 to 30 carbon atoms or a hydrogen atom,
R² is identical or different and represents a hydrogen atom or a monovalent, optionally substituted hydrocarbon radical having 1 to 20 carbon atoms,
Q represents an amine-substituted radical of formula
-R³-[NR⁴-R⁵-]ₓNR⁴₂ (II)
in which
R³ is identical or different and represents a divalent Si-C-bonded hydrocarbon radical having 1 to 18 carbon atoms,
R⁴ represents a hydrogen atom or a monovalent linear, cyclic or branched, saturated or unsaturated hydrocarbon radical having 1-18 carbon atoms,
R⁵ is identical or different and represents a divalent hydrocarbon radical having 1 to 6 carbon atoms,
x is 0 or an integer from 1 to 10,
k is an integer from 10 to 1000,
l is an integer from 1 to 100 and
y is 0 or 1, preferably 0,
are employed.

3. Aqueous emulsions according to Claim 1 or 2, **characterized in that** (D) cationic emulsifiers are co-used.

4. Aqueous emulsions according to Claim 1, 2 or 3, **characterized in that** as auxiliaries (E) preservatives, pH regulators, silanes, rheology additives, disinfectants, wetting agents, corrosion inhibitors, colorants or fragrances are employed.

5. Use of the aqueous emulsions according to any of Claims 1 to 4 for producing cosmetic formulations, in particular hair treatment compositions.

6. Use of the aqueous emulsions according to any of Claims 1 to 4 for producing washing and cleaning compositions.

## Revendications

1. Émulsions aqueuses, constituées par :
(A) 25 à 45 % en poids d'organopolysiloxanes à fonction amino, qui contiennent des radicaux reliés à Si-C avec de l'azote basique,
(B) 0 à 5 % en poids d'organopolysiloxanes, qui sont différents des organopolysiloxanes à fonction amino (A),
(C) 10 à 20 % en poids d'émulsifiants non ioniques,
(D) 0 à 5 % en poids d'émulsifiants ioniques,
(E) 0 à 2 % en poids d'adjuvants et
(F) 40 à 65 % en poids d'eau,
en tant qu'émulsifiants non ioniques (C), des éthers de polyéthylène glycols d'alcools primaires linéaires, qui comprennent des radicaux hydrocarbonés linéaires d'au plus 10 atomes de carbone, de la formule:
CH₃-(CH₂)ₘ-O-(CH₂-CH₂-O)ₚ-R⁶ (VI),
dans laquelle R⁶ signifie un atome d'hydrogène ou un radical hydrocarboné en C₁₋₄,
m signifie un nombre entier de 5 à 9, et
p signifie un nombre entier de 3 à 20,
étant utilisés,
à condition que l'utilisation d'éthers de polyéthylène glycols d'alcools primaires qui comprennent des radicaux hydrocarbonés ramifiés et d'éthers de polyéthylène glycols d'alcools secondaires soit exclue.

2. Émulsions aqueuses selon la revendication 1, **caractérisées en ce qu'**en tant qu'organopolysiloxanes à fonction amino (A), ceux de la formule :
R*_{3-y}Q_{y}SiO[R₂SiO]ₖ[RQSiO]ₗSiQ_{y}R*_{3-y} (III),
dans laquelle
R* signifie R ou un radical de la formule -OR²,
les R sont identiques ou différents, et signifient un radical hydrocarboné monovalent, éventuellement substitué, de 1 à 30 atomes de carbone, ou un atome d'hydrogène,
les R² sont identiques ou différents, et signifient un atome d'hydrogène ou un radical hydrocarboné monovalent, éventuellement substitué, de 1 à 20 atomes de carbone,
Q signifie un radical substitué avec des groupes amino de la formule:
-R³-[NR⁴-R⁵-]ₓNR⁴₂ (II)
dans laquelle
les R³ sont identiques ou différents, et signifient un radical hydrocarboné bivalent, relié à SiC, de 1 à 18 atomes C,
R⁴ signifie un atome d'hydrogène ou un radical hydrocarboné monovalent linéaire, cyclique ou ramifié, saturé ou insaturé, de 1 à 18 atomes de carbone,
les R⁵ sont identiques ou différents, et signifient un radical hydrocarboné bivalent de 1 à 6 atomes C,
x signifie 0 ou un nombre entier de 1 à 10,
k signifie un nombre entier de 10 à 1 000,
l signifie un nombre entier de 1 à 100, et
y signifie 0 ou 1, de préférence 0, sont utilisés.

3. Émulsions aqueuses selon la revendication 1 ou 2, **caractérisées en ce que** (D) des émulsifiants cationiques sont utilisés.

4. Émulsions aqueuses selon la revendication 1, 2 ou 3, **caractérisées en ce qu'**en tant qu'adjuvants (E), des conservateurs, des agents pour l'ajustement du pH, des silanes, des additifs de rhéologie, des désinfectants, des agents mouillants, des inhibiteurs de corrosion, des colorants ou des parfums sont utilisés.

5. Utilisation des émulsions aqueuses selon l'une quelconque des revendications 1 à 4 pour la fabrication de formulations cosmétiques, notamment d'agents de traitement des cheveux.

6. Utilisation des émulsions aqueuses selon l'une quelconque des revendications 1 à 4 pour la fabrication de détergents.
